# EUROPEAN PATENT APPLICATION

(11) **EP 4 537 860 A1**
(43) Date of publication of application: **16.04.2025**
(21) Application number: 23819737.0
(22) Date of filing: 31.05.2023
(51) Int. Cl.: A61L 27/12, A61C 5/70, A61C 8/00, A61L 27/02, A61L 27/04, A61L 27/06

(54) **DENTAL MATERIAL**

(30) Priority: 10.06.2022 JP 2022094264
(71) Applicant: Toagosei Co., Ltd., Minato-ku Tokyo 105-8419 (JP)
(72) Inventor: UESUGI, Yuuki, Nagoya-shi, Aichi 455-0026 (JP); YAMADA, Yoshinao, Nagoya-shi, Aichi 455-0026 (JP)
(74) Representative: Rüger Abel Patentanwälte PartGmbB
(86) International application number: PCT/JP2023/020388
(87) International publication number: WO 2023/238757

(57) **Abstract**

A dental material according to the disclosure contains zinc-supporting zirconium phosphate in which zinc ions are supported on zirconium phosphate.

## Description

### Technical Field

The present disclosure relates to a dental material.

### Background Art

Conventionally, a material having excellent biocompatibility (for example, titanium, a titanium alloy, zirconia, or the like) has been used as an implant material.

Unlike natural teeth, bacteria easily enter between an implant and the mucosa. Since a conventional implant material does not have an antibacterial action, a bacterium may adhere to the surface of an implant. As a result, peri-implantitis may occur. Peri-implantitis may cause an implant to fall out of the bone.

Patent Literature 1 discloses an antibacterial dental resin capable of preventing adhesion and propagation of bacteria. The resin disclosed in Patent Literature 1 contains an antibacterial agent at a mass ratio of from 0.2 to 10%. Patent Literature 1 discloses an antibacterial agent in which silver or both silver and zinc are supported on a support made of zirconium phosphate as the antibacterial agent. Patent Literature 1 discloses that the antibacterial dental resin is used for a crown.

Patent Literature 2 discloses an oral hygiene product composition that does not change its antibacterial activity even by heat treatment and the presence of water. The composition disclosed in Patent Literature 2 is obtained by blending an antibacterial ceramic in which an antibacterial metal is supported on a ceramic as a medicinal material. Patent Literature 2 discloses an antibacterial ceramic in which zinc is supported on a ceramic made of zirconium phosphate as the antibacterial ceramic. Patent Literature 2 specifically discloses a toothpaste, a powder toothpaste, a mouthwash, an application agent, and oral pasta as an oral hygiene product.

### Citation List

### Patent Literature

Patent Literature 1: Japanese Patent Application Laid-Open (JP-A) No. H08-119821
Patent Literature 2: JP-A No. H06-122617

### SUMMARY OF INVENTION

### Technical Problem

However, silver ions contained in the resin disclosed in Patent Literature 1 are unstable against exposure to heat and light, and are easily reduced to metallic silver. As a result, a crown formed using the resin disclosed in Patent Literature 1 is likely to be discolored, and aesthetics may be impaired.

Patent Literature 2 does not describe or suggest application to a dental material (for example, an implant material or the like) used for dental treatment and disposed in the oral cavity for a long period of time.

In view of the above circumstances, an object of the disclosure is to provide a dental material that has an antibacterial property and is hardly discolored.

### Solution to Problem

Specific means for solving the problem include the following aspects.
<1> A dental material, containing zinc-supporting zirconium phosphate in which zinc ions are supported on zirconium phosphate.
<2> The dental material according to <1>, in which the zirconium phosphate has a NASICON-type crystal structure or an α-type crystal structure.
<3> The dental material according to <1> or <2>, having an antibacterial property against at least one of a dental caries bacterium *(Streptococcus mutans)* or an oral bacterium *(Porphyromonas gingivalis).*
<4> The dental material according to any one of <1> to <3>, in which a supported amount of the zinc ions is 3 mass% or more with respect to a total amount of the zinc-supporting zirconium phosphate.
<5> The dental material according to any one of <1> to <4>, in which a supported amount of silver ions in the zinc-supporting zirconium phosphate is 1 mass% or less.
<6> The dental material according to any one of <1> to <5>, further containing
   at least one of titanium oxide or zirconium oxide, or a resin,
   wherein the dental material is used as a material of an implant.

### Advantageous Effects of Invention

According to the disclosure, a dental material which has an antibacterial property and is hardly discolored is provided.

### DESCRIPTION OF EMBODIMENTS

In the disclosure, a numerical range indicated using "to" means a range including numerical values described before and after "to" as a lower limit value and an upper limit value, respectively.

In the disclosure, the amount of each component in a composition means, if there are a plurality of substances corresponding to each component in the composition, a total amount of the plurality of substances present in the composition unless otherwise specified.

In the numerical ranges described in stages in the disclosure, the upper limit value or the lower limit value described in one numerical range may be replaced with the upper limit value or the lower limit value of a numerical range described in another stage. In a numerical range described in the disclosure, the upper limit value or the lower limit value of the numerical range may be replaced with a value shown in an example.

In the disclosure, a combination of preferred embodiments is a more preferred embodiment.

In the notation of a group (atomic group) in the disclosure, the notation which does not indicate substitution or non-substitution includes those without substituents as well as those with a substituent.

### (1) Dental Material

A dental material according to the disclosure contains zinc-supporting zirconium phosphate in which zinc ions are supported on zirconium phosphate.

In the disclosure, the "dental material" refers to a general material of a dental product that is used for dental treatment and is disposed in the oral cavity for a long period of time (for example, one or more days). Examples of the dental material include an implant material, a coronal material (for example, a crown resin, an artificial tooth, or the like), a dental filling material (for example, a composite resin, a root canal filling material, a bonding material, or the like), a dental adhesive material (for example, resin cement, orthodontic adhesive material, or the like), a fissure sealant, a coating material, a crown/bridge inlay resin, a pedestal construction material, a denture base resin, and a denture base repair resin. The dental material does not include a material of an article that is not disposed in the oral cavity for a long period of time (for example, an oral hygiene product described in Patent Literature 2, or the like).

The description "zinc ions are supported on zirconium phosphate" indicates a the supported amount of zinc ions is 0.1 mass% or more with respect to a total amount of the zinc-supporting zirconium phosphate.

A method of measuring the supported amount of zinc ions is similar to the method described in examples.

The dental material of the disclosure has the above configuration, and therefore has an antibacterial property and is hardly discolored. As a result, a bacterium hardly adheres to a dental product formed using the dental material of the disclosure. As a result, the dental material of the disclosure can, for example, prevent the onset of inflammation such as peri-implantitis, and can maintain excellent aesthetics of the dental product.

This effect is presumed to be due to the following reasons, but is not limited thereto.

Zinc ions are relatively stable to exposure to heat and light and are hardly reduced to metallic zinc. Furthermore, zinc ions exhibit an antibacterial action. As a result, it is presumed that the dental material of the disclosure has an antibacterial property and is hardly discolored.

The form of the dental material is not particularly limited, and is appropriately selected according to the application or the like of the dental material, and examples thereof include a solid material (for example, a powder, a tablet, or the like), a semi-solid material (for example, a gel, a paste, or the like), and a liquid material (for example, a solution, a dispersion, or the like).

### (1.1) Zinc-Supporting Zirconium Phosphate

The dental material contains zinc-supporting zirconium phosphate.

The zinc-supporting zirconium phosphate has zirconium phosphate and zinc ions supported on the zirconium phosphate.

The form of the zinc-supporting zirconium phosphate is not particularly limited, and is, for example, a particle.

The content of the zinc-supporting zirconium phosphate is not particularly limited, and is appropriately selected according to the application or the like of the dental material, and is preferably from 0.001 mass% to 70 mass%, and more preferably from 0.01 mass% to 50 mass% with respect to a total amount of the dental material.

### (1.1.1) Zirconium Phosphate

Zirconium phosphate has excellent biocompatibility and functions as a support for zinc ions.

The form of the zirconium phosphate is not particularly limited, and is, for example, a particle.

The raw material of the zirconium phosphate is, for example, a powder of zirconium phosphate (hereinafter referred to as "zirconium phosphate powder").

The median diameter of the zirconium phosphate powder is not particularly limited, and is appropriately selected according to the application or the like of the dental material, and is preferably from 0.1 µm to 5 µm, more preferably from 0.1 µm to 4 µm, still more preferably from 0.2 µm to 3 µm, and particularly preferably from 0.3 µm to 2 µm.

Considering the processability into a dental product, not only the median diameter but also the maximum particle diameter of the zirconium phosphate powder is important. The maximum particle diameter of the zirconium phosphate powder is not particularly limited, and is appropriately selected according to the application or the like of the dental material, and is preferably 10 µm or less, more preferably 6 µm or less, and still more preferably 4 µm or less.

The median diameter is measured on a volume basis using a laser diffraction particle size distribution.

The crystal structure of the zirconium phosphate is not particularly limited, and may be amorphous or crystalline (for example, an α-type crystal structure, a β-type crystal structure, a γ-type crystal structure, a NASICON-type crystal structure, or the like.).

Among these, the zirconium phosphate preferably has a NASICON-type crystal structure or an α-type crystal structure from the viewpoint of improving the antibacterial property of the dental material, or the like.

Hereinafter, the zirconium phosphate having an α-type crystal structure is also referred to as "α-zirconium phosphate". The zirconium phosphate having a NASICON-type crystal structure is also referred to as "NASICON-type zirconium phosphate".

### (1.1.2) Zinc Ions

Zinc ions exhibit an antibacterial action.

The supported amount of the zinc ions is not particularly limited, and is preferably 3 mass% or more with respect to the total amount of the zinc-supporting zirconium phosphate. As a result, the antibacterial property of the dental material is improved.

The supported amount of the zinc ions is more preferably from 3 mass% to 30 mass%, still more preferably from 3 mass% to 20 mass%, and particularly preferably from 4 mass% to 20 mass% with respect to the total amount of the zinc-supporting zirconium phosphate from the viewpoint of further improving the antibacterial property of the dental material, or the like.

A method of measuring the supported amount of zinc ions is similar to the method described in examples.

A method of allowing zirconium phosphate to support zinc ions is not particularly limited, and examples thereof include a method in which zinc ions are exchanged in zirconium phosphate, followed by thermal decomposition. Examples of a method of exchanging zinc ions include a method of immersing zirconium phosphate in an aqueous solution containing zinc sulfate.

### (1.1.3) Other Metal Ions

On the zirconium phosphate of the zinc-supporting zirconium phosphate, other metal ions may be supported in addition to zinc ions, or other metal ions need not be supported as long as the effects of the disclosure are not impaired from the viewpoint of improving the antibacterial property of the dental material, or the like.

The description "other metal ions are not supported" indicates that a supported amount of other ions is 0.1 mass% or less with respect to the total amount of the zinc-supporting zirconium phosphate, and includes 0 mass%.

A method of measuring the supported amount of other ions is similar to the method of measuring a supported amount of silver ions described in examples.

The other metal ion is at least one selected from the group consisting of a silver ion, a copper ion, a tin ion, an iron ion, a cobalt ion, a nickel ion, a manganese ion, and a barium ion.

In particular, silver ions may be further supported on the zirconium phosphate of the zinc-supporting zirconium phosphate from the viewpoint of further improving the antibacterial property of the dental material, or the like. When silver ions are further supported on the zirconium phosphate, the supported amount of silver ions is preferably reduced. Since the supported amount of silver ions is reduced, discoloration of the dental material can be prevented. In an embodiment of the present invention, the supported amount of silver ions is preferably 1 mass% or less, and more preferably 0.5 mass% or less with respect to the total amount of the zinc-supporting zirconium phosphate. Silver ions need not be supported on the zirconium phosphate. Furthermore, it is preferred that the content of silver is also reduced throughout the entire dental material, and the content of silver in the dental material is preferably 5 mass% or less, more preferably 1 mass% or less, and particularly preferably 0.5 mass% or less.

### (1.2) Metal, Ceramic, and Resin

The dental material may contain at least one of a metal, a ceramic, or a resin according to the application or the like of the dental material.

Each of the metal, the ceramic, and the resin may be a known material used as a dental product.

It is preferred that the dental material further contains at least one of titanium oxide or zirconium oxide, or a resin, and is used as a material of an implant. As a result, the dental material can prevent adhesion of a bacterium to the implant and can prevent occurrence of discoloration of the implant.

The implant includes an implant body to be fixed to the bone, an abutment detachable from the implant body, and an artificial crown fixed to the abutment. As a material of the implant body and the abutment, titanium oxide, zirconium oxide, titanium, a titanium alloy, or the like is generally used from the viewpoint of biocompatibility or the like. As a material of the artificial crown, a resin for an artificial crown or the like is generally used. Examples of the resin for an artificial crown include polyacrylate, polymethacrylate, polyolefin, polyamide, polyester, polyether, polynitrile, polyvinyl, a cellulose resin, a fluororesin, an imide resin, and a silicone resin.

When the dental material contains zinc-supporting zirconium phosphate and a resin, the dental material is suitably used as a material of an artificial crown. Examples of the resin include the above-mentioned resin for an artificial crown.

When the dental material contains zinc-supporting zirconium phosphate and at least one of titanium oxide or zirconium oxide, at least one of titanium oxide or zirconium oxide is suitably used for the material of the abutment and the implant body, and the zinc-supporting zirconium phosphate is suitably used for the material of an antibacterial film. The antibacterial film covers at least part of the surface of at least one of the abutment or the implant body. The antibacterial film may contain a known component different from the zinc-supporting zirconium phosphate according to a method of forming the antibacterial film described later, or the like. The antibacterial film is usually mainly formed of a metal component and does not contain a resin. The content of the resin in the dental material used as the antibacterial film may be 20 mass% or less, and is preferably 5 mass% or less.

A method of forming the antibacterial film is not particularly limited, and may be a known method, and examples thereof include a dry process and a wet process.

In the dry process, the antibacterial film is formed by a treatment in a gas phase or vacuum. Specifically, examples of the dry process include a physical vapor deposition method (for example, vacuum deposition, ion plating, sputtering, or the like), a chemical vapor deposition method (for example, thermal CVD (Chemical Vapor Deposition), plasma CVD, plasma CVD, or the like), hot dip coating, thermal spraying (for example, flame spraying, arc spraying, plasma spraying, or the like), lining, and a dry process surface modification method (for example, plasma surface modification, ion implantation, or the like).

In the wet process, the antibacterial film is formed using a solution. Specifically, examples of the wet process include plating (for example, electroplating, electroless plating, electroforming, or the like), electrolytic polymerization, a sol-gel method, a Langmuir project method, painting, and a wet process surface modification method (for example, anodization, chemical conversion treatment, or the like).

### (1.3) Additives

The dental material may contain an additive as necessary from the viewpoint of improving kneading processability into a resin and other physical properties, or the like.

Examples of the additive include a pigment, an inorganic ion exchanger (for example, zeolite or the like), a dye, an antioxidant, a light stabilizer, a flame retardant, an antistatic agent, a foaming agent, an impact strengthening agent, a glass fiber, a lubricant (for example, metal soap or the like), a moisture-proof agent, an extender, a coupling agent, a nucleating agent, a fluidity improver, a deodorant, a wood powder, an antifungal agent, an antifouling agent, a rust inhibitor, a metal powder, an ultraviolet absorber, and an ultraviolet shielding agent.

### (1.4) Target Bacteria

Bacteria on which the dental material exhibits an antibacterial effect include *Escherichia coli* and *Staphylococcus aureus.*

The dental material preferably has an antibacterial property against at least one of a dental caries bacterium *(Streptococcus mutans)* or an oral bacterium (*Porphyromonas gingivalis*). Thus, a dental caries bacterium and an oral bacterium hardly adhere to the dental material. As a result, the dental material can prevent the occurrence of dental caries and can further prevent the onset of inflammation such as peri-implantitis.

### Examples

Hereinafter, the disclosure will be described more specifically with reference to examples, but the disclosure is not limited to the following examples unless it goes beyond the gist of the disclosure.

### Production of Zirconium Phosphate

### Production Example 1

### (Production of α-Zirconium Phosphate)

Into a 2 L round-bottom flask, 1,345 mL of deionized water and 135 g of 35% hydrochloric acid were placed, 225 g of a 20 mass% aqueous solution of zirconium oxychloride octahydrate containing 0.18 mass% of hafnium was added thereto, and then 93 g of oxalic acid dihydrate was added and dissolved therein. While this solution was well stirred, 101 g of 75 mass% phosphoric acid was added thereto. This was heated to 98°C over 2 hours, and then refluxed for 12 hours with stirring. After cooling the reaction system, the obtained precipitate was collected by filtration, washed well with water, and then dried at 105°C under normal pressure to obtain first zirconium phosphate. This was crushed with a rotor speed mill (16,000 rpm (revolutions per minute), sieve opening: 80 µm).

Powder X-ray diffraction of the first zirconium phosphate was measured under the following conditions. As a result, it was verified that the first zirconium phosphate was α-zirconium phosphate having an α-type crystal structure.

### Production Example 2

### (Production of NASICON-type Zirconium Phosphate)

Into a 2 L round-bottom flask, 1,000 mL of deionized water was placed, 366 g of a 20 mass% aqueous solution of zirconium oxychloride octahydrate containing 0.18 mass% of hafnium was added thereto, and then 38 g of oxalic acid dihydrate was added and dissolved therein. While this solution was well stirred, 123 g of 75 mass% phosphoric acid was added thereto. Subsequently, 15 mass% aqueous ammonia was added dropwise thereto until the pH reached 3, and this was heated to 98°C over 2 hours, and then refluxed for 24 hours with stirring. After cooling the reaction system, the obtained precipitate was collected by filtration, washed well with water, and then dried at 105°C under normal pressure to obtain ammonium-type zirconium phosphate having a NASICON-type crystal structure. A powder obtained by crushing this with a rotor speed mill (16,000 rpm, sieve opening 80 µm) was heated to 750°C over 4 hours in an electric muffle furnace, and then fired for 10 hours. Thus, second zirconium phosphate was obtained.

Powder X-ray diffraction of the obtained second zirconium phosphate was measured under the following conditions. As a result, it was verified that the second zirconium phosphate was proton-type zirconium phosphate having a NASICON-type crystal structure (hereinafter referred to as "NASICON-type zirconium phosphate").

### Powder X-Ray Diffraction

As an X-ray diffractometer, "D8 ADVANCE" manufactured by BRUKER was used. An X-ray diffraction diagram was obtained using CuKα generated at an applied voltage of 40 kV and a current value of 40 mA using a Cu-sealed X-ray source. Detailed measurement conditions are as follows.

### [1.3.1] Measurement Conditions

X-ray source: sealed X-ray source (Cu line source), 0.4 × 12 mm², Long Fine Focus
Rating: 2.2 kW
Use output: 40 kV-40 mA (1.6 kW)
Goniometer radius: 280 mm
Sample stage: FlipStick_Twin_Twin-XE
Measurement range 2θ: from 5° to 55°
Step width: 0.02°
Step time: 0.05 seconds/step
Incident side solar slit: 2.5°
Anti-scattering slit: 10.5 mm
Curvature: 1.00
Detector: LYNXEYE XE
Detector slit width: 5.758 mm
Detector window width: 2.9°

### Dental Material

### Example 1

### (Introduction of Zn Ions into α-Zirconium Phosphate)

In a 500 mL beaker, 5 g of the α-zirconium phosphate obtained in Production Example 1, 41.7 g of zinc sulfate heptahydrate, and 453 g of pure water were placed, stirred with a stirrer, stirred at 100°C for 24 hours, followed by filtration. Thereafter, filtration washing was performed until the electric conductivity of the filtrate reached 100 µS/cm or less, and the resulting powder was dried at 120°C for 2 hours under normal pressure and pulverized in an agate mortar. As a result, a first powder (hereinafter, also referred to as "Zn-substituted α-zirconium phosphate") (zinc-supporting zirconium phosphate) was obtained.

The mass of the first powder was measured. The first powder was dissolved in a mixed liquid of nitric acid and hydrofluoric acid, and the Zn concentration and the Zr concentration were measured by ICP emission spectrometry. From these measurement results, Zn (supported amount of zinc ions) introduced into the α-zirconium phosphate was 17 mass% with respect to a total amount of the Zn-substituted α-zirconium phosphate.

The Zn-substituted α-zirconium phosphate (1 g) prepared by the above method, 0.1 g of sodium hexametaphosphate, 10 g of pure water, and 2 g of an acrylic emulsion ("NW-7090" manufactured by Toagosei Company, Limited) were placed and stirred with a stirrer to obtain a dispersion (dental material).

This dispersion was applied onto a PET (polyethylene terephthalate) plate using a bar coater No. 28 and dried at 80°C for 5 minutes to obtain a plate for an antibacterial test.

### Example 2

### (Introduction of Zn Ions into NASICON-Type Zirconium Phosphate)

In a 100 mL beaker, 5 g of the NASICON-type zirconium phosphate obtained in Production Example 2, 1.6 g of zinc nitrate hexahydrate, and 100 g of pure water were placed, stirred with a stirrer, then evaporated to dryness at 100°C. Thereafter, the resultant was pulverized in an agate mortar and fired at 750°C for 4 hours. As a result, a second powder (hereinafter, also referred to as "Zn-substituted NASICON-type zirconium phosphate") (zinc-supporting zirconium phosphate) was obtained.

The mass of the second powder was measured. The second powder was dissolved in a mixed liquid of nitric acid and hydrofluoric acid, and the Zn concentration and the Zr concentration were measured by ICP emission spectrometry. From these measurement results, Zn (supported amount of zinc ions) introduced into the NASICON-type zirconium phosphate was 7 mass% with respect to the total amount of the Zn-substituted NASICON-type zirconium phosphate.

A dispersion (dental material) and a plate for an antibacterial test were produced under the same conditions as in Example 1 except that the Zn-substituted NASICON-type zirconium phosphate prepared by the above method was changed to the Zn-substituted α-zirconium phosphate of Example 1.

### Comparative Example 1

### (No Zinc-Supporting Zirconium Phosphate)

A dispersion (dental material) and a plate for an antibacterial test were produced under the same conditions as in Example 1 except that the Zn-substituted α-zirconium phosphate of Example 1 was not used.

### Comparative Example 2

### (Introduction of Ag Ions into NASICON-Type Zirconium Phosphate)

In a 100 mL beaker, 5 g of the NASICON-type zirconium phosphate obtained in Production Example 2, 0.6 g of silver nitrate, and 100 g of pure water were placed, stirred at 60°C for 2 hours, followed by filtration. Thereafter, filtration washing was performed until the electric conductivity of the filtrate reached 100 µS/cm or less, and the resulting powder was dried at 120°C for 2 hours under normal pressure and pulverized in an agate mortar, and then fired at 750°C for 4 hours. As a result, a third powder (hereinafter, also referred to as "Ag-substituted NASICON-type zirconium phosphate") was obtained.

The mass of the third powder was measured. The third powder was dissolved in a mixed liquid of nitric acid and hydrofluoric acid, and the Ag concentration and the Zr concentration were measured by ICP emission spectrometry. From these measurement results, Ag (supported amount of silver ions) introduced into the NASICON-type zirconium phosphate was 3 mass% with respect to the total amount of the Ag-substituted NASICON-type zirconium phosphate.

A dispersion (dental material) and a plate for an antibacterial test were produced under the same conditions as in Example 1 except that the Zn-substituted α-zirconium phosphate of Example 1 was changed to the Ag-substituted NASICON-type zirconium phosphate.

### Evaluation

### Antibacterial Test

The viable cell counts of *Escherichia coli, Staphylococcus aureus,* an oral bacterium, and a dental caries bacterium in the plates for an antibacterial test were measured according to the method described in JIS Z 2801.

### Light Resistance Test

A light resistance test in which the plate for an antibacterial test was irradiated with a sunshine carbon arc lamp for 8 hours was performed, the state of discoloration of the plate for an antibacterial test was visually observed, and evaluation was performed according to the following criteria. The results are shown in Table 1.

The acceptable rating for the light resistance test is "A".
A: Discoloration of the plate for an antibacterial test after the light resistance test could not be observed as compared to before the light resistance test.
B: Discoloration of the plate for an antibacterial test after the light resistance test was observed as compared to before the light resistance test.

**[Table 1]**

| | Dental material | | | Antibacterial test | | | | Light resistance test |
|---|---|---|---|---|---|---|---|---|
| | Zinc-supporting zirconium phosphate | | | *E. coli* | *S.aureus* | *P.gingivalis* | *S. mutans* | Evaluation |
| | Zinc ions | Silver ions | Zirconium phosphate | Viable cell count | Viable cell count | Viable cell count | Viable cell count | |
| | Supported amount | Supported amount | Crystal structure | | | | | |
| | (mass%) | (mass%) | | (cells/cm²) | (cells/cm²) | (cells/cm²) | (cells/cm²) | |
| Example 1 | 17 | 0 | α-type | 30 | 17 | 0 | 0 | A |
| Example 2 | 7 | 0 | NASICON-type | 340 | < 0.06 | 0 | 0 | A |
| Comparative Example 1 | - | - | - | 380000 | 2100 | 18000 | 4400 | A |
| Comparative Example 2 | 0 | 3 | NASICON-type | 0 | 0 | 0 | 0 | B |

In Table 1, *"E. coli"* stands for *Escherichia coli. "S. aureus"* stands for *Staphylococcus aureus.* "*P. gingivalis"* indicates an oral bacterium (*Porphyromonas gingivalis*)*.* "*S. mutans*" indicates a dental caries bacterium *(Streptococcus mutans).*

The dental material of Comparative Example 1 did not contain zinc-supporting zirconium phosphate. Therefore, the viable cell count of *Escherichia coli* was 380,000 cells/cm². The viable cell count of *Staphylococcus aureus* was 2,100 cells/cm². The viable cell count of the oral bacterium was 18,000 cells/cm². The viable cell count of the dental caries bacterium was 4,400 cells/cm².

In Comparative Example 2, the dental material contained the Ag-substituted NASICON-type zirconium phosphate, but did not contain zinc-supporting zirconium phosphate. Therefore, the viable cell count of *Escherichia coli* was 0 cells/cm². The viable cell count of *Staphylococcus aureus* was 0 cells/cm². The viable cell count of the oral bacterium was 0 cells/cm². The viable cell count of the dental caries bacterium was 0 cells/cm². The evaluation result of the light resistance test was "B".

From these results, it was found that the dental material of Comparative Example 2 has an antibacterial property and is hardly discolored.

The dental materials of Examples 1 and 2 contained zinc-supporting zirconium phosphate in which zinc ions are supported and silver ions are not supported on zirconium phosphate. Therefore, the viable cell count of *Escherichia coli* was 30 cells/cm² or less. The viable cell count of *Staphylococcus aureus* was 17 cells/cm² or less. The viable cell count of the oral bacterium was 0 cells/cm². The viable cell count of the dental caries bacterium was 0 cells/cm². That is, each of the viable cell count of *Escherichia coli,* and the viable cell counts of *Staphylococcus aureus,* the oral bacterium, and the dental caries bacterium in Examples 1 and 2 was decreased as compared to Comparative Example 1. The evaluation result of the light resistance test was "A".

From these results, it was found that the dental materials of Examples 1 and 2 have an antibacterial property and are hardly discolored.

The disclosure of Japanese Patent Application No. 2022-094264 filed on June 10, 2022 is incorporated herein by reference in its entirety.

All documents, patent applications, and technical standards described in this description are incorporated herein by reference to the same extent as if each individual document, patent application, and technical standard were specifically and individually indicated to be incorporated by reference.

## Claims

1. A dental material, comprising zinc-supporting zirconium phosphate in which zinc ions are supported on zirconium phosphate.

2. The dental material according to claim 1, wherein the zirconium phosphate has a NASICON-type crystal structure or an α-type crystal structure.

3. The dental material according to claim 1, having an antibacterial property against at least one of a dental caries bacterium *(Streptococcus mutans)* or an oral bacterium *(Porphyromonas gingivalis).*

4. The dental material according to claim 1, wherein a supported amount of the zinc ions is 3 mass% or more with respect to a total amount of the zinc-supporting zirconium phosphate.

5. The dental material according to claim 1, wherein a supported amount of silver ions in the zinc-supporting zirconium phosphate is 1 mass% or less.

6. The dental material according to any one of claims 1 to 5, further comprising:
at least one of titanium oxide or zirconium oxide, or a resin,
wherein the dental material is used as a material of an implant.
